# EUROPEAN PATENT SPECIFICATION

(11) **EP 3 362 105 B1**
(45) Date of publication and mention of the grant of the patent: **03.12.2025**
(21) Application number: 15787036.1
(22) Date of filing: 13.10.2015
(51) Int. Cl.: A61L 2/14, A61L 2/22, B05B 13/04, B05B 12/12

(54) **STERILIZATION SYSTEM**
STERILISATIONSSYSTEM
SYSTÈME DE STÉRILISATION

(43) Date of publication of application: 22.08.2018
(73) Proprietor: Suntory Holdings Limited, Osaka-shi, Osaka 530-8203 (JP); Plasmatreat GmbH, 33803 Steinhagen (DE)
(72) Inventor: HIGASHIYAMA, Kenichi, Kyoto 619-0284 (JP); TOMINAGA, Kenta, Kyoto 619-0284 (JP); HIRAYAMA, Yuji, Kyoto 619-0284 (JP); YOSHIHARA, Kazuki, Kyoto 619-0284 (JP); IIZUKA, Toshiaki, Tokyo 135-8631 (JP); MORIYA, Satoshi, Tokyo 135-8631 (JP); BUSKE, Christian, 33803 Steinhagen (DE); HASSE, Daniel, 33803 Steinhagen (DE)
(74) Representative: Mewburn Ellis LLP
(86) International application number: PCT/JP2015/005186
(87) International publication number: WO 2017/064741

(56) References cited:
- US-A- 4 129 092
- US-A1- 2005 217 701
- US-A1- 2013 142 694
- US-A1- 2013 319 460
- US-A1- 2014 311 095

## Description

### Technical Field

This disclosure relates to a sterilization system including a sterilizing device having a nozzle to spray sterilizing agent onto a sterilization object and a conveying unit conveying a plurality of sterilization objects one after another continuously to the sterilizing device.

### Background Art

As a method of sterilizing a sterilization object, a sterilizing method is known from Japanese Unexamined Patent Application Publication No. 2013-28398 (Patent Literature 1). or US2014311095.

Patent Literature 1 describes a method of sterilizing an interior of a container by blowing (spraying) an amount of a sterilizing agent containing hydrogen peroxide into a container as the sterilization object.

When the above-described sterilizing method is applied to the above-noted sterilization system, a following problem can occur. Namely, sterilization of the container requires blowing of an amount of the sterilizing agent required for the sterilization into the container. However, in the case of the sterilization system configured to convey sterilization objects continuously to a sterilizing device, it may occur that the required amount of sterilizing agent may not be blown onto the sterilization object which has passed under a nozzle for spraying the sterilizing agent. Thus, there is the possibility of the sterilization of the sterilization object being insufficient.

### Citation List

### Patent Literature

PTL 1: Japanese Unexamined Patent Application Publication No. 2013-28398.

### Summary of Invention

### Technical Problem

Accordingly, there remains a need for a sterilization system capable of conveying sterilization objects to a sterilizing device continuously, yet capable of sterilizing the sterilization objects sufficiently.

### Solution to Problem

The present invention relates to a sterilization system defined according to the claims.

A sterilization system according to this disclosure comprises:
a sterilizing device having a nozzle to spray sterilizing agent onto a sterilization object; and
a conveying unit conveying a plurality of sterilization objects one after another continuously to the sterilizing device;
the nozzle being capable of moving a reaching position of the sterilizing agent sprayed therefrom to the conveying unit along a conveying path of the sterilization objects; and
a controlling unit executing a following control which causes the reaching position to follow each sterilization object as being conveyed by synchronizing a moving speed of the reaching position of the sterilizing agent with a conveying speed of the sterilization object.

With the above-described arrangement, the reaching position of the sterilizing agent to the conveying unit is caused to follow the sterilization object as being conveyed. Accordingly, it is possible to extend the period of spraying the sterilizing agent onto the sterilization object. Consequently, it becomes possible to spray a sufficient amount of sterilizing agent onto the sterilization objects while these sterilization objects are being conveyed continuously to the sterilizing device, so that the sterilization objects can be sterilized sufficiently.

Next, the sterilization system relating to this disclosure will be described by way of preferred embodiments thereof. It is understood, however, that the scope of the present disclosure is not to be limited to these preferred embodiments.

In the case of using sterilizing agent containing hydrogen peroxide as in the sterilizing method described in Patent Document 1, a cleaning operation needs to be effected after the use of the agent so that any of the sterilizing agent will not remain on the sterilization object. However, elimination of entire sterilizing agent is difficult with such cleaning operation. Thus, there is a risk of some of the sterilizing agent remaining on the sterilization object. Further, in the case of the sterilizing method of Patent Document 1, the spraying operation of the sterilizing agent and the cleaning operation subsequent thereto are carried out mainly at a high temperature. Thus, if the sterilization object is made of a material that suffers thermal contraction, e.g. a resin material, various restrictions will be imposed on the sterilizing process if excessive thermal contraction or deformation is to be avoided. For this reason, according to the present invention:
the nozzle includes a plasma generating section for generating plasma, an outlet section for spraying the sterilizing agent, and a relaying section for feeding the plasma generated by the plasma generating section to the outlet section;
the sterilizing device includes a water vapor feeding section for feeding water vapor to the relaying section; and
the outlet section sprays, as the sterilizing agent, the plasma fed from the plasma generating section and ROS (Reactive Oxidizing Species) generated through a reaction between the plasma and the water vapor fed from the water vapor feeding section. Namely, the sterilization of the sterilization object can be effected with using the sterilizing agent containing the plasma and the ROS. As ROS disappears (disintegrates) with lapse of time, there occurs no residual thereof. Further, sterilization using ROS does not require application of such heat that may cause thermal contraction in the sterilization object. Therefore, the above-described problems of residual of sterilizing agent and thermal contraction/deformation of the sterilization object can be avoided.

According to one configuration, preferably, the ROS contains as its principal component hydroxy radical. With the presence of hydroxy radical which has a particularly high reactivity among the various ROS's, an even higher sterilization effect can be achieved.

When the spray opening is caused to follow the sterilization object, the amount of sterilizing agent required for the sterilization object will be greater when this sterilization object is large and so will be the distance (following distance) along which the spray opening is caused to follow the sterilization object. And, if the following distance is to be increased, this will lead to enlargement and increased complexity of the device configuration needed for the following control. For this reason, according to a further configuration, the sterilization object comprises a cap to be attached to a bottle. Namely, a cap to be attached to a bottle is relatively small. Thus, as this is employed as the sterilization object thereby to decrease the required following distance, it becomes possible to achieve side reduction and simplification of the device configuration required for the following control.

According to the present invention:
the outlet section is provided in the form of a circular plate having a center axis extending through the respective centers of circular top and bottom faces thereof, the center axis being oriented in a horizontal direction, the outlet section having in its lateral face a spray opening for spraying the sterilizing agent, the outlet section being rotatable about the center axis;
the conveying path, in its region where the sterilization object is sterilized by the sterilizing device, is disposed downwardly of the outlet section and extends in the horizontal direction perpendicular to the center axis;
the reaching position of the sterilizing agent sprayed to the conveying unit is movable along the conveying path in association with rotating of the outlet section; and the controlling unit executes the following control through controlling of a moving speed of the reaching position by way of controlling of a rotating speed of the outlet section.

With the above, with the above-described simple arrangement of configuring an outlet section provided in the form of a circulate plate rotatable about its center axis as the rotation axis, it is possible to cause its spray opening to follow the sterilization object.

According to a further configuration:
the outlet section has in its lateral face a plurality of the spray openings;
the conveying unit conveys the respective sterilization objects one after another by intervals corresponding to spacing between the respective spray openings; and the controlling unit executes the following control for the respective spray openings, with associating the plurality of spray openings with the respective sterilization objects one by one in order.

With the above arrangement, it becomes possible to increase the number of sterilization objects that can be sterilized in a unit period, by a degree corresponding to the increase in the number of spray openings.

In the case of the arrangement of the conveying path of the conveying unit being disposed downwardly of the outlet section, the sterilizing agent will not be fed directly to the sterilization object at least while the spray opening is located upwardly of the rotation axis (in other words, while the opening is located in the upper half of the circulate plate-like outlet section), so the sterilizing agent will be wasted. Then, according to one configuration, the spray openings are configured not to spray the sterilizing agent while being located upwardly of the center axis. With this, wasteful feeding of the sterilizing agent can be restricted.

Further and other technical advantageous effects and features of this disclosure will become apparent upon reading the following explanation of exemplary and nonlimiting embodiments with reference to the accompanying drawings.

### Brief Description of Drawings

[fig.1] Fig. 1 is a schematic configuration diagram showing a sterilizing device for effecting ROS sterilization according to a first embodiment.
[fig.2]Fig. 2 is a block diagram showing the sterilizing device for effecting ROS sterilization according to the first embodiment.
[fig.3]Fig. 3 is a schematic front view showing a sterilization system according to the first embodiment.
[fig.4]Fig. 4 is a schematic side view showing the sterilization system according to the first embodiment.,
[fig.5]Fig. 5 shows states of the sterilization system according to the first embodiment wherein a reaching position of sterilizing agent follows a sterilization object.
[fig.6]Fig. 6 is a schematic front view showing a sterilization system according to a second embodiment.
[fig.7]Fig. 7 shows conditions of a sterilization system according to a third embodiment wherein a reaching position of sterilizing agent follows a sterilization object.

### Description of Embodiments

### First Embodiment

Next, a first embodiment of a sterilization system relating to this disclosure will be explained with reference to the accompanying drawings. In the sterilization system of this embodiment, the system effects ROS sterilization with using reactive oxidizing species (ROS, e.g. OH radial, singlet oxygen, etc.) generated with using plasma, as a sterilizing agent for sterilizing a sterilization object 80. Then, a device configuration of a sterilizing device 1 for effecting the ROS sterilization will be explained.

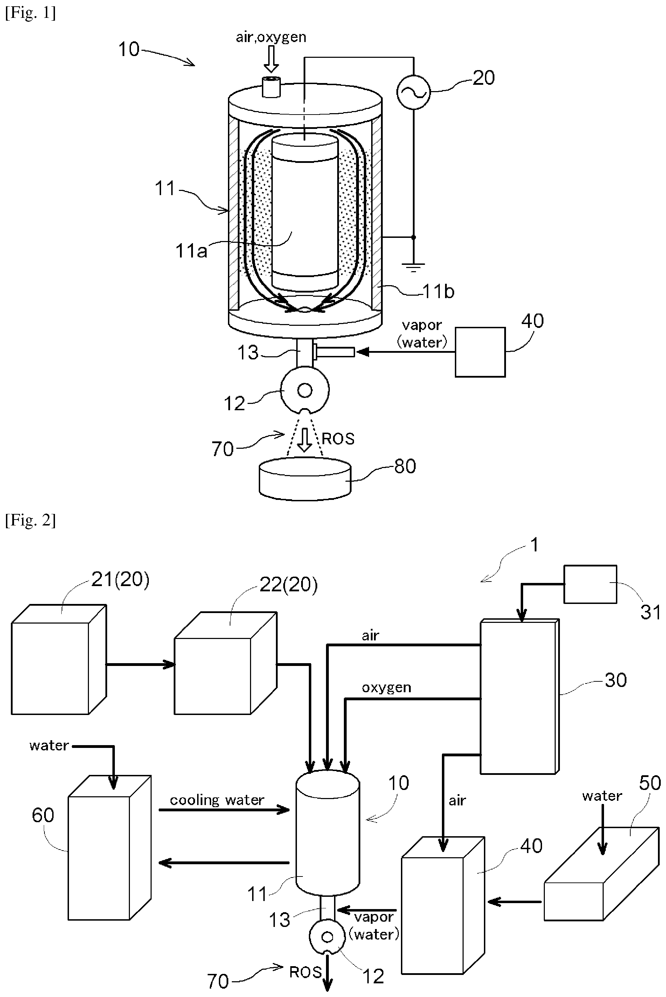

Fig. 1 shows an ROS generating configuration or mechanism implemented by the sterilizing device 1. The sterilizing device 1 includes a nozzle 10 for generating ROS and spraying (irradiating) this ROS together with plasma as a sterilizing agent 70 to the sterilization object 80. This nozzle 10 includes a plasma generating section 11 for generating plasma, an outlet section 12 for spraying the sterilizing agent 70, and a relaying section 13 coupled to the plasma generating section 11 and the outlet section 12.

This nozzle 10 is configured to generate so-called atmospheric pressure plasma as the plasma. The use of atmospheric pressure plasma allows device cost reduction since such component as a vacuum vessel required otherwise for low pressure plasma can be omitted, and allows also a continuous operation, thus achieving high operational efficiency. The use of atmospheric pressure plasma provides a further advantage that as this can be generated even at a low temperature, exposure of the sterilization object to high temperature can be avoided. Next, there will be explained generation of such atmospheric pressure plasma (to be referred to simply as "plasma" hereinafter) by the nozzle 10 and the ROS generation utilizing the plasma.

The plasma generating section 11 having a well-known configuration includes an internal electrode 11a and an external electrode 11b. With this plasma generating section 11 in operation, by applying a high voltage (e.g. an effective voltage of 20 kV at frequency of 14 kHz) between the internal electrode 11a and the external electrode 11b, an electric field is generated within this plasma generating section 11 by an AC power source 20. And, gas together with air is introduced into the plasma generating section 11 to pass this gas through the generated electric field, whereby plasma is generated. The generated plasma is fed to the relaying section 13. In the instant embodiment, oxygen (O2) is introduced as an example of the gas to the plasma generating section 11, so that this oxygen is plasmatized. This plasmatization results in generation of oxygen radical and ozone (O3), which are then fed to the relaying section 13.

The relaying section 13 is connected to an evaporator (an example of "water vapor feeding unit" to be detailed later) 40 to receive also water vapor therefrom. With this, in the relaying section 13, there occurs a reaction between the plasma fed from the plasma generating section 11 (oxygen radical and ozone) and water vapor fed from the evaporator 40, thereby to generate ROS. In the instant embodiment, it is provided such that through the reaction between oxygen plasma (oxygen radical and ozone) and water vapor, there is mainly generated hydroxy radical ( • OH) which has a particularly high reactivity among various kinds of ROS.

More particularly, in association with the reaction between water vapor and plasma, hydrogen radical (H) and hydroxy radical (OH) are produced from H₂O according to the following formula (1).

H₂O → • H + • OH............. (1)

Further, as the hydrogen radical reacts with ozone, there are produced hydroxy radical and oxygen (O2) according to the following formula (2).

H + O₃→ • OH + O₂...... (2)

Then, the above formula (1) and formula (2) can be integrated into a formula (3) as follows.

H₂O + O₃→ 2 • OH + O₂....... (3)

Namely, through the reaction between oxygen plasma and water vapor, the reaction of the above formula (3) is caused, so that hydroxy radical ( • OH) can be generated in an efficient manner. As a result, the ROS thus produced contains highly reactive hydroxy radical as its principal component. With presence of hydroxy radical as its principal component, high sterilization effect can be achieved. And, as this ROS thus produced together with any plasma unreacted with the water vapor is sprayed as a sterilizing agent 70 from the outlet section 12 onto the sterilization object 80, sterilization of this sterilization object 80 is effected.

Next, the device configuration of the sterilizing device 1 shown in Fig.2 for effecting the ROS sterilization will be explained. The sterilizing device 1 includes, in addition to the nozzle 10, a generator 21 and a transformer 22 which together constitute the AC power source 20, a gas feeding unit 30 for feeding various kinds of gas to the nozzle 10 and the evaporator 40, the evaporator 40 for feeding water vapor to the relaying section 13, a pump 50 for feeding liquid water to the evaporator 40, and a chiller 60 for feeding cooling water to the nozzle 10.

The generator 21 generates an alternating current. For instance, in this embodiment, there is employed such current having a frequency of 14 kHz, an effective voltage of 300 V and an effective current of 11A. Then, the voltage of the AC current provided by the generator 21 is boosted from 300 V to 20 kV by the transformer 22. With this, a high voltage of 20 kV is applied between the internal electrode 11a and the external electrode 11b of the plasma generating section 11.

The gas feeding unit 30 feeds oxygen (O₂) together with air to the nozzle 10. The gas feeding unit 30 also feeds air which is used for feeding water vapor generated by the evaporator 40 to the relaying section 13. The gas feeding unit 30 includes a control panel 31. By operating this control panel 31, feeding amounts of the various gas to respective destinations can be adjusted. In the instant embodiment, it is configured in particular such that with an operation of the control panel 31, air is fed by 6L/min and oxygen is fed by 3L/min to the nozzle 10 and also air is fed by 3 L/min to the evaporator 40.

The evaporator 40 is configured such that a heating coil (not shown) incorporated therein is heated to 300°C, and liquid water fed from the pump 50 is heated by this heating coil to generate water vapor. Then, the water vapor is mixed with the air fed from the gas feeding unit 30, and the water vapor together with the air are fed to the relaying section 13. Incidentally, in the instant embodiment, the pump 50 can be configured to feed water by 1.2 mL/min to the evaporator 40, for instance.

The chiller 60 is configured such that with supply of cooling water to the nozzle 10, this nozzle 10 heated with the application of the high voltage thereto is cooled (chilled).

With the above-described sterilizing device 1 in operation, oxygen fed together with air from the gas feeding unit 30 to the nozzle 10 is plasmatized by the plasma generating section 11 and the oxygen plasma thus generated is caused to react in the relaying section 13 with the water vapor fed together with air from the evaporator 40, whereby ROS containing hydroxy radical as its principal component is produced in a continuous manner. And, as the ROS produced continuously in the relaying section 13 and any plasma unreacted with the water vapor is sprayed continuously as the sterilizing agent 70 from the outlet section 12, continuous treatment of the sterilization object 80 is made possible. In the instant embodiment, the sterilizing agent 70 containing plasma and ROS is sprayed by a flow rate of 50000 mm/sec at a temperature ranging from 50 to 80°C from the outlet section.

Sterilization using ROS provides the following advantages. In the case of using a sterilizing agent containing hydrogen peroxide, it is necessary to effect a post cleaning operation to ensure that no sterilizing agent will remain on the sterilization object. However, it is difficult to remove all sterilizing agent by cleaning so there is a risk of some sterilizing agent remaining on the sterilization object inadvertently. Moreover, the spraying operation of the sterilizing agent and the cleaning operation subsequent thereto are carried out mainly at a high temperature. Thus, if the sterilization object is made of a material that suffers thermal contraction, e.g. a resin material, various restrictions will be imposed on the sterilizing process if excessive thermal contraction or deformation is to be avoided. On the other hand, as ROS disappears (disintegrates) with lapse of time, there occurs no residual thereof. Further, sterilization using ROS does not require application of such heat that may case thermal contraction in the sterilization object. Therefore, the above-described problems of residual of sterilizing agent and thermal contraction/deformation of the sterilization object can be avoided.

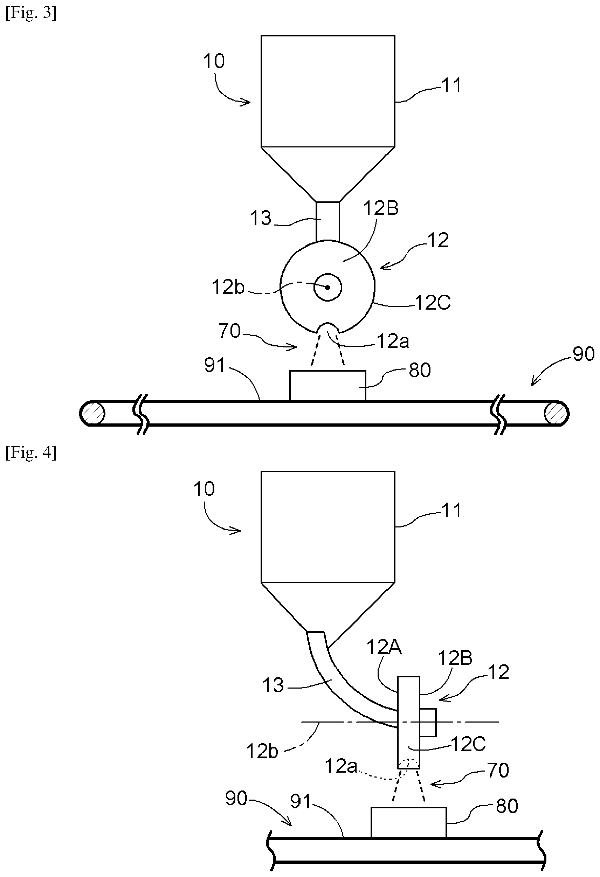

The sterilization system relating to this disclosure, as shown in Fig. 3, includes the sterilizing device 1 having the nozzle 10 for spraying the above-described sterilizing agent 70 onto the sterilization object 80 (illustration of other components than the nozzle 10 is omitted in Fig. 3), and a conveying unit 90 for conveying a plurality of sterilization objects 80 one after another in a continuous manner to the sterilizing device 10.

In the case of the sterilization system relating to this embodiment, in order to ensure a longer period for spraying (irradiating) the sterilizing agent 70 containing plasma and ROS onto the sterilization objects which are being conveyed continuously by the conveying unit 90, the nozzle 10 is capable of moving a reaching position of the sprayed sterilizing agent 70 to the conveying unit 90 along a conveying path 91 of the sterilization objects 80. Further, an unillustrated controlling section executes a following control that causes the reaching position 71 to follow the sterilization objects 80 by synchronizing the moving speed of the reaching positon 71 with the conveying speed of the sterilization objects 80 conveyed by the conveying unit 90. Next, with reference to Figs. 3, 4 and 5, one possible configuration for realizing the above will be explained.

As may be apparent from Fig. 3 and Fig. 4, the outlet section 12 is provided in the form of a circular plate. And, a center axis 12b extending through the respective centers of a circular bottom face 12A and top face 12B extends in a horizontal direction. The outlet section 12 includes, in its lateral face 12C, a spray opening 12a for spraying the sterilizing agent 70. Moreover, the outlet section 12 is configured to be rotatable about the center axis 12b as the rotation axis. And, the conveying path 91 of the conveying unit 90 is disposed downwardly of the outlet section 12 in a region thereof where the sterilization object 80 is to be sterilized by the sterilizing device 1 and extends in the horizontal direction perpendicular to the center axis 12b.

With the above-described configuration, firstly, in association with rotation of the outlet section 12, the spray opening 12a provided in the lateral face 12C turns around the center axis 12b. With this, the spraying direction of the sterilizing agent 70 from the outlet section 12 varies. In the course of this, when the sterilizing agent 70 is being sprayed from the spray opening 12a, as the outlet section 12 is rotated clockwise by 180 degrees from a state wherein the spray opening 12a is located on the right side as seen in its front view (the angle shown in Fig. 3) (that is, as the spray opening 12a is turned on the lower side of the center axis 12b), the trajectory of the reaching position of the sterilizing agent 70 sprayed from the spray opening 12a to the horizontal plane downwardly of the outlet section 12 will be aligned with a line normal to the center axis 12b. That is, the trajectory of the reaching position of this sterilizing agent 70 will be in alignment, under the outlet section 12, with the conveying path 91 of the conveying unit 90 which path extends in the horizontal direction perpendicular to the center axis 12b. Therefore, the reaching position of the sterilizing agent 80 sprayed from the outlet section 12 to the conveying path 91 is movable (displaceable) along the conveying path 91 in association with rotation of the outlet section 12. Therefore, if the outlet section 12 is rotated in accordance with a conveying speed of the sterilization object 80 as being conveyed on the conveying path 91, the reaching position of the sterilizing agent 70 to the conveying unit 90 can be caused to follow the sterilization object 80. In this way, according to the instant embodiment, with the simple arrangement of the outlet section 12 provided in the form of a circular plate being configured to be rotatable about its center axis 12b as the rotation axis, it has been made possible to cause this spray opening 12a to follow the sterilization object 80.

Incidentally, when the spray opening 12a is caused to follow the sterilization object 80 which is being conveyed, if this sterilization object 80 is large, the amount of sterilizing agent 70 required for its sterilization will be greater, so that the distance ("following distance") along which the spray opening 12a is caused to follow the sterilization object 80 too will be greater. And, if the following distance is to be increased, this will lead to increase in size and complexity of the device configuration required for the following, disadvantageously. On the other hand, in the case of the instant embodiment, as the sterilization object 80, there is employed a cap to be attached to a PET bottle. Namely, such cap to be attached to a bottle is relatively small. Then, by using this as the sterilization object 80, the required following distance is made shorter, whereby the device configuration required for the following can be made smaller and simpler. Incidentally, the above described use is just exemplary and the object to be used as the sterilization object 80 is not particularly limited.

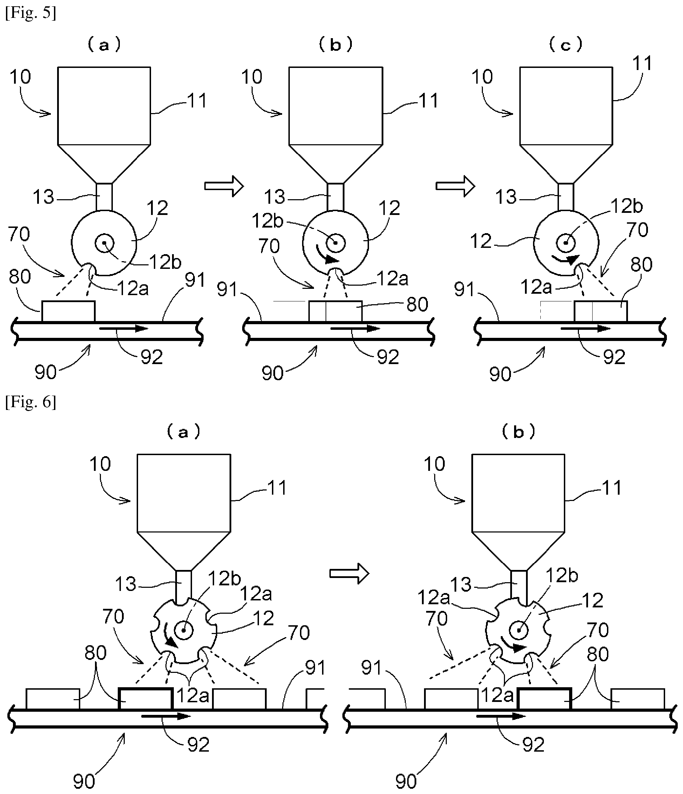

And, in order to cause the reaching position of the sterilizing agent 70 to follow the sterilization object 80, the controlling unit controls the moving speed of the reaching position of the sterilizing agent 70 through control of the rotational speed of the outlet section 12 in such a manner as to synchronize the moving speed of the reaching position of the sterilizing agent 70 with the conveying speed of the sterilization object 80. For instance, referring to Figs. 5a through 5c, as shown in Fig. 5a, suppose that the spray opening 12a is currently located at a desired position on the proximal side in a conveying direction 92 of the sterilization object 80 relative to the center axis 12b and the sterilizing agent 70 is being sprayed from the spray opening 12a located at this desired position toward the sterilization object 80. Then, from this state, the controlling unit, as the following control, will adjust the rotational speed of the outlet section 12 (or start rotation of the outlet section 12) which is being rotated, in such a manner to synchronize the moving speed of the reaching position of the rotated outlet section 12 with the conveying speed of the sterilization object 80. With this, as shown in Fig. 5b, 5c, the reaching position of the sterilizing agent 70 (in other words, the spray opening 12a) is caused to follow the sterilization object 80.

While the reaching position of the sterilizing agent 70 is being caused to follow the sterilization object 80, the sterilizing agent 70 will be fed continuously to this sterilization object 80. With this, it is possible to increase the period of feeding the sterilizing agent 70 to (onto/against) the sterilization object 80. Therefore, while the sterilization objects 80 can be conveyed continuously to the sterilizing device 1, it is possible to supply a sufficient amount of the sterilizing agent 70 to the sterilization object 80, so that this sterilization object 80 can be sterilized sufficiently.

As methods of the following control, following methods are conceivable. Firstly, suppose that the sterilization objects 80 are conveyed by equal intervals. In this case, the outlet section 12 will be set to a constant rotation state and also a following range for causing the reaching position of the sterilizing agent 70 to follow the sterilization object 80 will be set in advance. Then, the controlling unit will cause the outlet section 12 to rotate in such a manner that for each sterilization object 80, when this sterilization object 80 enters the following range, the spray opening 12a will be located at a position corresponding thereto and also until the sterilization object 80 exits the following range, the reaching position of the sterilizing agent 70 (in other words the spray opening 12a) may follow the sterilization object 80. In this, the controlling unit may vary the rotational speed of the outlet section 12 during one whole rotation of this outlet section 12 or can rotate the outlet section 12 at an equal speed if possible.

Further, in this, it is preferred that the spraying of the sterilizing agent 70 from the spray opening 12a be effected in an intermittent manner. For instance, it is preferred that while the spray opening 12a is located on the upper side of the center axis 12b (in other words, in the upper half of the circular plate-like outlet section 12), no sterilizing agent 70 be sprayed. With this, at least while the spray opening 12a is located upwardly of the center axis 12b, there will occur no direct spraying of the sterilizing agent 70 from this spray opening 12a toward to the sterilization object 80. Thus, no waste of sterilizing agent 70 will occur and such waste can be avoided advantageously. More preferably, an arrangement is provided such that the spray opening 12a sprays the sterilizing agent 70 only when the sterilization object 80 is present within the following range. Incidentally, the intermittent spraying of the sterilizing agent 70 can be realized by a mechanical arrangement to be provided within the outlet section 12 (for instance, an arrangement that the sterilizing agent 70 is sprayed from the spray opening 12a only within a certain positional range) and can be effected based on result of position detection of the spray opening 12a and/or the sterilization object 80 by a sensor.

In addition to the above, alternative following control as follows is conceivable. With rotation of the outlet section 12 being stopped, the spray opening 12a will be disposed in advance at a desired position on the proximal side in the conveying direction 92 relative to the center axis 12b. And, only when an unillustrated sensor detects that the sterilization object 80 has been conveyed to the reaching position of the sterilizing agent 70 at the desired position, rotation of the outlet section 12 and spraying of the sterilizing agent 70 are started and spraying of the sterilizing agent 70 to the sterilization object 80 is continued with causing the reaching position of the sterilizing agent 70 to follow the sterilization object 80. And, when the unillustrated sensor detects that the sterilization object 80 has been conveyed to a desired position set on the distal side in the conveying direction 92 relative to the center axis 12b, rotation of the outlet section 12 and spraying of the sterilizing agent are stopped, thus stopping the following and sterilization on the sterilization object 80. Thereafter, the spray opening 12a is returned again to the above-described desired position and the process waits ready for conveyance of the next sterilization object 80. Alternatively, the following control can be effected with continuous detection of the position of the sterilization object 80 by the sensor. Incidentally, the above described method of following control is just exemplary and the following control is not limited thereto.

### Second Embodiment

Next, a second embodiment of a sterilization system relating to the present disclosure will be described with reference to the accompanying drawing. In this embodiment, the configuration of the outlet section 12 differs from that of the first embodiment. Next, the sterilization system relating to the this embodiment will be explained with emphasis being mainly put on the differences from the first embodiment. Incidentally, as for those respects not explicitly described, such respects are understood to be identical to those of the first embodiment. And, they are provided with same or similar reference marks and detailed discussion thereof will be omitted.

In the instant embodiment, as shown in Fig. 6, the outlet section 12 includes a plurality of (five in the illustrated embodiment) spray openings 12a in its lateral face 12C. And, these spray openings 12a are disposed with equal spacing therebetween on the lateral face 12C. The conveying unit 90 is configured to convey the respective sterilization objects 80 one after another by the intervals corresponding to the spacing of the respective spray openings 12a. And, the controlling unit executes a following control for the respective spray openings 12a, with associating the plurality of spray openings 12a with the respective sterilization objects 80 one by one in order. More particularly, each spray opening 12a is assigned to each one of the sterilization objects 80 which are conveyed one after another; and the controlling section rotates the outlet section 12 in such a manner as to cause each reaching position of the sterilizing agent 70 sprayed from the spray opening 12a to follow the assigned sterilization object 80. With the above arrangement, it becomes possible to increase the number of sterilization objects 80 that can be sterilized in a unit period, in comparison with the first embodiment having only one spray opening 12a.

### Reference Third Embodiment

Next, a reference third embodiment of a sterilization system relating to the present disclosure will be described with reference to the accompanying drawing. In this reference embodiment, the configuration of the outlet section 12 differs from those of the first and second embodiments. Next, the sterilization system relating to this embodiment will be explained with emphasis being mainly put on the differences from the first embodiment. Incidentally, as for those respects not explicitly described, such respects are understood to be identical to those of the first embodiment. And, they are provided with same or similar reference marks and detailed discussion thereof will be omitted.

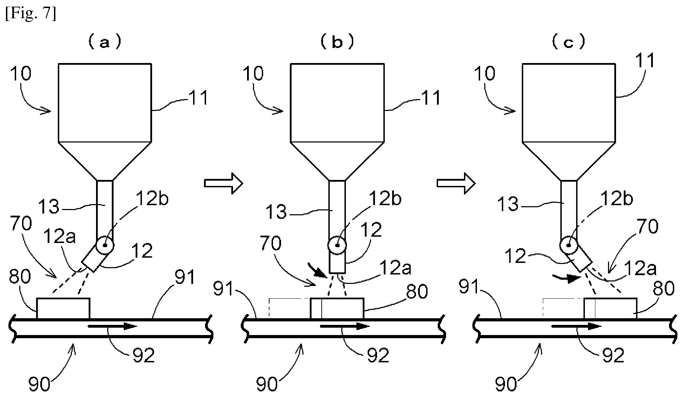

In the instant reference embodiment, as shown in Fig. 7, the outlet section 12 is provided in the form of a pipe pivotally connected to the relaying section 13, with one end of the pipe being rotatable (or pivotable) about a rotation axis 12b oriented in the horizontal direction. And, like the first embodiment the conveying path 91 of the conveying unit 90 is disposed downwardly of the outlet section 12 in the range where the sterilization object 80 is to be sterilized by the sterilizing device 1 and extends along the horizontal direction normal to the rotation axis 12b. Therefore, like the first embodiment, by rotating (pivoting) the outlet section 12 in accordance with a conveying speed of the sterilization object 80 conveyed on the conveying path 91, it is possible to cause the reaching position of the sterilizing agent 70 to the conveying unit 90 to follow the sterilization object 80. The method of following control is not particularly limited. For instance, a following control method similar to the following control described in the first embodiment can be adopted within possible range.

### Other Embodiments

Lastly, other reference embodiments of the sterilization system relating to this disclosure will be described. In the foregoing embodiment, there was explained the exemplary configuration in which the outlet section 12 is rotatably (pivotally) connected to the relaying section 13 and the reaching position of the sterilizing agent 70 is caused to follow the sterilization object 80 by rotating (pivoting) the spray opening 12a about the center axis (rotation axis) 12b. However, the reference embodiment of the present disclosure is not limited thereto. For instance, the outlet section 12 or the nozzle 10, either one thereof as a whole, can be configured to be moved in parallel with the sterilization object 80. Further, the sterilization system relating to this disclosure can be applied also to a sterilization system using a sterilizing agent other than the sterilizing agent containing ROS and plasma.

As for the other configurations too, the embodiments disclosed in this disclosure are understood to be exemplary in all respects, and it is understood that the scope of this disclosure is not to be limited thereto. Those skilled in the art will easily understand that various modifications will be possible within a range not deviating from the essential concept of this disclosure.

### Industrial Applicability

This disclosure is applicable to a sterilization system for sterilizing a sterilization object, for instance.

### Reference Signs List

1 sterilizing device
10 nozzle
11 plasma generating section
12 outlet section
12A bottom face
12B top face
12C lateral face
12a spray opening
12b center axis
13 relaying section
40 evaporator
70 sterilizing agent
80 sterilization object
90 conveying unit
91 conveying path

## Claims

1. A sterilization system comprising:
a sterilizing device (1) having a nozzle (10) to spray sterilizing agent onto sterilization objects and having a water vapor feeding section (40) for feeding water vapor to a relaying section (13); and
a conveying unit (90) for conveying a plurality of sterilization objects one after another continuously to the sterilizing device (1) along a conveying path (91);
the nozzle (10) including a plasma generating section (11) for generating plasma, an outlet section (12) for spraying the sterilizing agent, the relaying section (13) for feeding the plasma generated by the plasma generating section to the outlet section (12), and being capable of moving a reaching position of the sterilizing agent sprayed therefrom to the conveying unit (90) along the conveying path (91) of the sterilization objects; and
a controlling unit to execute a following control which causes the reaching position of the sterilizing agent to follow each sterilization object as being conveyed, by synchronizing a moving speed of the reaching position of the sterilizing agent with a conveying speed of the sterilization object,
wherein:
the outlet section (12) is provided in the form of a circular plate having a center axis (12b) extending through the respective centers of circular top and bottom faces thereof, the center axis (12b) being oriented in a horizontal direction, the outlet section (12) having a spray opening (12a) for spraying the sterilizing agent in a lateral face (12C) thereof, the outlet section (12) being rotatable about the center axis (1b);
the conveying path (91), in its region where the sterilization object is sterilized by the sterilizing device (1), is disposed downwardly of the outlet section (12) and extends in the horizontal direction perpendicular to the center axis (12b);
the reaching position of the sterilizing agent sprayed to the conveying unit (90) is movable along the conveying path (91) in association with rotating of the outlet section (12);
the controlling unit executes the following control through controlling of a moving speed of the reaching position by way of controlling of a rotating speed of the outlet section (12); and
whereby the outlet section (12) sprays, as the sterilizing agent, the plasma fed from the plasma generating section (11) and ROS (reactive oxidizing species) generated through reaction between the plasma and the water vapor fed from the water vapor feeding section (40).

2. The sterilization system according to claim 1, wherein:
the outlet section (12) has in its lateral face (12C) a plurality of the spray openings (12a);
the conveying unit (90) conveys the respective sterilization objects one after another by intervals corresponding to spacing between the respective spray openings (12a); and
the controlling unit executes the following control for the respective spray openings (12a), with associating the plurality of spray openings with the respective sterilization objects one by one in order.

3. The sterilization system according to claim 1 or 2, wherein the or each spray opening (12a) is configured not to spray the sterilizing agent while located upwardly of the center axis (12b).

## Patentansprüche

1. Sterilisationssystem, umfassend:
eine Sterilisationsvorrichtung (1), die eine Düse (10) zum Sprühen eines Sterilisationsmittels auf Sterilisationsgegenstände aufweist und einen Wasserdampfzufuhrabschnitt (40) zum Zuführen von Wasserdampf zu einem Weiterleitungsabschnitt (13) aufweist; und
eine Fördereinheit (90) zum Befördern einer Vielzahl von Sterilisationsgegenständen kontinuierlich nacheinander zur Sterilisationsvorrichtung (1) entlang eines Förderwegs (91);
wobei die Düse (10) einen Plasmaerzeugungsabschnitt (11) zum Erzeugen von Plasma, einen Auslassabschnitt (12) zum Sprühen des Sterilisationsmittels, den Weiterleitungsabschnitt (13) zum Zuführen des Plasmas, das durch den Plasmaerzeugungsabschnitt erzeugt wurde, zu dem Auslassabschnitt (12) umfasst und die dazu in der Lage ist, eine Auftreffposition des Sterilisationsmittels, das aus dieser auf die Fördereinheit (90) entlang des Förderwegs (91) der Sterilisationsgegenstände gesprüht wird, zu bewegen; und
eine Steuereinheit zum Ausführen einer Nachfolgesteuerung, die bewirkt, dass die Auftreffposition des Sterilisationsmittels jedem Sterilisationsgegenstand, wie er befördert wird, folgt, indem eine Bewegungsgeschwindigkeit der Auftreffposition des Sterilisationsmittels mit einer Fördergeschwindigkeit des Sterilisationsgegenstands synchronisiert wird,
wobei:
der Auslassabschnitt (12) in Form einer kreisförmigen Platte mit einer Mittelachse (12b) bereitgestellt ist, die sich durch die entsprechenden Mittelpunkte einer kreisförmigen oberen und unteren Fläche desselben erstreckt, wobei die Mittelachse (12b) in eine horizontale Richtung ausgerichtet ist, wobei der Auslassabschnitt (12) eine Sprühöffnung (12a) zum Sprühen des Sterilisationsmittels in einer lateralen Fläche (12C) desselben aufweist, wobei der Auslassabschnitt (12) um die Mittelachse (1b) drehbar ist;
der Förderweg (91) in dessen Bereich, in dem der Sterilisationsgegenstand durch die Sterilisationsvorrichtung (1) sterilisiert wird, unterhalb des Auslassabschnitts (12) angeordnet ist und sich in die horizontale Richtung normal auf die Mittelachse (12b) erstreckt;
die Auftreffposition des Sterilisationsmittels, das auf die Fördereinheit (90) gesprüht wird, entlang des Förderwegs (91) unter Zuordnung der Drehung des Auslassabschnitts (12) bewegbar ist;
die Steuereinheit die Nachfolgesteuerung durch die Steuerung einer Bewegungsgeschwindigkeit der Reichweitenposition ausführt, indem eine Drehzahl des Auslassabschnitts (12) gesteuert wird; und
wodurch der Auslassabschnitt (12) das Plasma, das von dem Plasmaerzeugungsabschnitt (11) zugeführt wurde, als das Sterilisierungsmittel und ROS (reaktive oxidierende Spezies), die durch Reaktion zwischen dem Plasma und dem Wasserdampf, der von dem Wasserdampfzufuhrabschnitt (40) zugeführt wurde, erzeugt wurden, sprüht.

2. Sterilisationssystem nach Anspruch 1, wobei:
der Auslassabschnitt (12) in dessen lateraler Fläche (12C) eine Vielzahl der Sprühöffnungen (12a) aufweist;
die Fördereinheit (90) die entsprechenden Sterilisationsgegenstände nacheinander in Abständen, die einer Beabstandung zwischen den entsprechenden Sprühöffnungen (12a) entsprechen, befördert; und
die Steuereinheit die Nachfolgesteuerung für die entsprechenden Sprühöffnungen (12a) unter Zuordnung der Vielzahl von Sprühöffnungen zu den entsprechenden Sterilisationsgegenständen der Reihe nach ausführt.

3. Sterilisationssystem nach Anspruch 1 oder 2, wobei die oder jede Sprühöffnung (12a) ausgelegt ist, um das Sterilisationsmittel nicht zu sprühen, während sie oberhalb der Mittelachse (12b) angeordnet ist.

## Revendications

1. Système de stérilisation comprenant :
un dispositif de stérilisation (1) ayant une buse (10) pour pulvériser un agent de stérilisation sur des objets à stériliser et ayant une section d'alimentation en vapeur d'eau (40) pour alimenter en vapeur d'eau une section de relais (13) ; et
une unité de transport (90) pour transporter une pluralité d'objets à stériliser, les uns après les autres de manière continue, vers le dispositif de stérilisation (1) le long d'un chemin de transport (91) ;
la buse (10) comportant une section de génération de plasma (11) pour générer du plasma, une section de sortie (12) pour pulvériser l'agent de stérilisation, la section de relais (13) pour alimenter la section de sortie (12) en plasma généré par la section de génération de plasma, et étant capable de déplacer une position de contact de l'agent de stérilisation pulvérisé par celle-ci sur l'unité de transport (90) le long du chemin de transport (91) des objets à stériliser ; et
une unité de commande pour exécuter une commande de suivi qui amène la position de contact de l'agent de stérilisation à suivre chaque objet à stériliser lorsqu'il est transporté, en synchronisant une vitesse de déplacement de la position de contact de l'agent de stérilisation avec une vitesse de transport de l'objet à stériliser,
dans lequel :
la section de sortie (12) est fournie sous la forme d'une plaque circulaire ayant un axe central (12b) s'étendant à travers les centres respectifs de faces circulaires supérieure et inférieure de celle-ci, l'axe central (12b) étant orienté dans une direction horizontale, la section de sortie (12) ayant une ouverture de pulvérisation (12a) pour pulvériser l'agent de stérilisation dans une face latérale (12C) de celle-ci, la section de sortie (12) pouvant tourner autour de l'axe central (1b) ;
le chemin de transport (91), dans sa région où l'objet à stériliser est stérilisé par le dispositif de stérilisation (1), est disposé en dessous de la section de sortie (12) et s'étend dans la direction horizontale perpendiculairement à l'axe central (12b) ;
la position de contact de l'agent de stérilisation pulvérisé vers l'unité de transport (90) est mobile le long du chemin de transport (91) en association avec la rotation de la section de sortie (12) ;
l'unité de commande exécute la commande de suivi par commande d'une vitesse de déplacement de la position de contact par commande d'une vitesse de rotation de la section de sortie (12) ; et
la section de sortie (12) pulvérisant ainsi, en tant qu'agent de stérilisation, le plasma alimenté par la section de génération de plasma (11) et des espèces oxydantes réactives (RCS) générées par réaction entre le plasma et la vapeur d'eau alimentée par la section d'alimentation en vapeur d'eau (40).

2. Système de stérilisation selon la revendication 1, dans lequel :
la section de sortie (12) possède dans sa face latérale (12C) une pluralité d'ouvertures de pulvérisation (12a) ;
l'unité de transport (90) transporte les objets à stériliser respectifs les uns après les autres par intervalles correspondant à un espacement entre les ouvertures de pulvérisation respectives (12a) ; et
l'unité de commande exécute la commande de suivi pour les ouvertures de pulvérisation respectives (12a), en associant la pluralité d'ouvertures de pulvérisation avec les objets à stériliser respectifs un par un dans l'ordre.

3. Système de stérilisation selon la revendication 1 ou 2, dans lequel l'ouverture ou chaque ouverture de pulvérisation (12a) est configurée pour ne pas pulvériser l'agent de stérilisation tandis qu'elle est située au-dessus de l'axe central (12b).
